# EUROPEAN PATENT SPECIFICATION

(11) **EP 4 495 639 B1**
(45) Date of publication and mention of the grant of the patent: **21.01.2026**
(21) Application number: 23306252.0
(22) Date of filing: 20.07.2023
(51) Int. Cl.: G02B 1/04, C07C 321/18, C07D 251/38

(54) **OPTICAL MATERIAL DERIVED FROM A POLYMERIZABLE COMPOSITION CONTAINING TERMINAL ALKYNE AND THIOL FUNCTIONS**
OPTISCHES MATERIAL AUS EINER POLYMERISIERBAREN ZUSAMMENSETZUNG MIT TERMINALEN ALKYN- UND THIOLFUNKTIONEN
MATÉRIAU OPTIQUE DÉRIVÉ D'UNE COMPOSITION POLYMÉRISABLE CONTENANT DES FONCTIONS TERMINAUX ALCYNE ET THIOL

(43) Date of publication of application: 22.01.2025
(73) Proprietor: Essilor International, 94220 Charenton-Le-Pont (FR)
(72) Inventor: FROMENTIN, Pierre, 10240 Bangkok (TH)
(74) Representative: Jacobacci Coralis Harle

(56) References cited:
- CN-B- 106 008 994
- MAVILA SUDHEENDRAN ET AL: "High Refractive Index Photopolymers by Thiol-Yne "Click" Polymerization", APPLIED MATERIALS & INTERFACES, vol. 13, no. 13, 29 March 2021 (2021-03-29), US, pages 15647 - 15658, XP093120662, ISSN: 1944-8244, DOI: 10.1021/acsami.1c00831
- ZHANG HONGLI ET AL: "Asymmetric Michael addition in an aqueous environment with the assistance of optically active hyperbranched polymers", POLYMER CHEMISTRY, vol. 8, no. 11, 1 January 2017 (2017-01-01), Cambridge, pages 1771 - 1777, XP093120455, ISSN: 1759-9954, DOI: 10.1039/C7PY00036G
- QIU YANG ET AL: "Photo-Curing Vis-IR Hybrid Fresnel Lenses with High Refractive Index", MACROMOLECULAR CHEMISTRY AND PHYSICS, vol. 222, no. 24, 29 September 2021 (2021-09-29), DE, XP093120691, ISSN: 1022-1352, Retrieved from the Internet <URL:https://onlinelibrary.wiley.com/doi/full-xml/10.1002/macp.202100311> DOI: 10.1002/macp.202100311
- COOK ALEXANDER B. ET AL: "Hyperbranched Polymers with High Degrees of Branching and Low Dispersity Values: Pushing the Limits of Thiol-Yne Chemistry", MACROMOLECULES, vol. 49, no. 4, 2 February 2016 (2016-02-02), US, pages 1296 - 1304, XP093120704, ISSN: 0024-9297, DOI: 10.1021/acs.macromol.6b00132

## Description

The present invention relates to optical materials having improved optical and mechanical properties, which can be used in particular in optical substrates such as ophthalmic lenses, having generally a middle or high refractive index. The present invention is also directed to polymerizable compounds and a method of making the optical material.

### BACKGROUND AND SUMMARY OF THE INVENTION

Plastic materials have been developed as alternatives and replacements for glass in applications such as optical lenses, fiber optics, windows, and automotive, nautical and aviation industries. As compared to inorganic glass, organic polymeric materials are advantageous in terms of light weight, high impact resistance, ease of molding and dyeability.

However, the refractive indices of many polymeric materials are lower than that of glass. Optically transparent plastic materials having a higher refractive index are of major interest since they allow the manufacture optical articles such as lenses of lower thickness for an equivalent corrective power.

Using thiol-ene chemistry, i.e., an organic reaction between a thiol and an alkene to form a thioether, polymers with both high glass transition temperature and high refractive indexes are often difficult to achieve with simple, cheap and commercially available monomers. For example, polymerization of a stoichiometric mixture of pentaerythritol tetrakis (mercaptopropionate) and pentaerythritol tetraacrylate provides a polymer having limited glass transition temperature (13°C) and refractive index (1.545). The known methods to improve the optical and mechanical properties of polymers obtained by thiol-ene reactions are not suitable, as they involve the use of very specific monomers that include highly polarizable bonds such as carbon-metal or phosphorus-chalcogens.

US 2016/376453 discloses a curable composition comprising a polythiol constituent, an alkene-containing and/or alkyne-containing constituent, and an epoxy-containing constituent. The polythiol constituent can derive from a mercaptan-containing terpene or terpenoid, a mercaptan-containing cyclic alkene, a mercaptan-containing polycyclic alkene, a linear alkene, a mercaptan-containing alkyne, a mercaptan-containing unsaturated fatty acid, a mercaptan-containing unsaturated fatty ester, or a mercaptan-containing polyalkene. The cured composition is used for making cell phone cases or expanded polystyrene foam. However, three different chemicals need to be synthesized then blended to obtain the polymerizable composition.

The article "Hyperbranched Polymers by Thiol-Yne Chemistry: From Small Molecules to Functional Polymers", D. Konkolewicz et al., J. Am. Chem. Soc. 2009, 131, 50, 18075-18077 describes the homopolymerization of monomers comprising one terminal alkyne function and one thiol function, such as propargyl 3-mercaptopropionate. A catalytic amount of photoinitiator and UV radiation are used to add two thiols across one alkyne bond at room temperature, resulting in hyperbranched polymers.

The article "Hyperbranched Polymers with High Degrees of Branching and Low Dispersity Values: Pushing the Limits of Thiol-Yne Chemistry", A. B. Cook et al, Macromolecules 2016, 49, 4, 1296-1304 discloses the homopolymerization of monomers comprising one terminal alkyne function and one thiol function, or the copolymerization of such monomers with multifunctional core molecules (poly-ene or poly-yne monomers), in the presence of photoinitiator and under UV irradiation, resulting in the formation of hyperbranched thiol-yne polymers.

The article "High Refractive Index Photopolymers by Thiol-Yne "Click" Polymerization", M. Sudheendran et al., ACS Appl. Mater. Interfaces, 2021, 13, 13, 15647-15658 describes high index polymers obtained by polymerization of polythiols with multifunctional alkynes.

Thus, there is a need in the art to develop a polymeric material having an adequate refractive index and good impact resistance/strength for practical use in optical articles, at a reasonable cost.

The present invention relates to an optical material having a refractive index higher than or equal to 1.50 obtained by polymerization of a polymerizable composition comprising at least one polythiol and at least one polymerizable compound of formula (I): 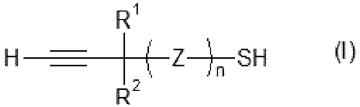 in which R¹ and R² represent, independently of each other, a hydrogen atom, a substituted or unsubstituted alkyl group, an aryl group, or R¹ and R², taken together, form a divalent group of formula -R¹-R²-, in which -R¹-R²- represents a substituted or unsubstituted alkylene group, Z represents a divalent group connected to the adjacent mercapto -SH group through a carbon atom, and n = 0 or 1.

The present invention provides highly crosslinked polymer networks from thiol-yne reactions, thanks to specifically designed starting materials copolymerizable with polythiols, and curable through photochemical processes. A same monomer molecule of formula (I) bears two different reactive functions that usually are introduced in a polymerizable composition through different molecules. In the present case, the starting materials feature both alkyne and thiol functions.

Another advantage of the present invention is that the starting monomers can be finely tuned by adapting the substitution pattern to control their mechanical properties and refractive indexes.

### DETAILED DESCRIPTION OF THE INVENTION

The terms "comprise" (and any grammatical variation thereof, such as "comprises" and "comprising"), "have" (and any grammatical variation thereof, such as "has" and "having"), "contain" (and any grammatical variation thereof, such as "contains" and "containing"), and "include" (and any grammatical variation thereof, such as "includes" and "including") are openended linking verbs. They are used to specify the presence of stated features, integers, steps or components or groups thereof, but do not preclude the presence or addition of one or more other features, integers, steps or components or groups thereof. As a result, a method, or a step in a method, that "comprises," "has," "contains," or "includes" one or more steps or elements possesses those one or more steps or elements, but is not limited to possessing only those one or more steps or elements.

Unless otherwise indicated, all numbers or expressions referring to quantities of ingredients, ranges, reaction conditions, etc. used herein are to be understood as modified in all instances by the term "about."

In the present description, unless otherwise specified, an optical article/material is understood to be transparent when the observation of an image through said optical article is perceived with no significant loss of contrast, that is, when the formation of an image through said optical article is obtained without adversely affecting the quality of the image. This definition of the term "transparent" can be applied to all objects qualified as such in the description, unless otherwise specified.

The optical material of the invention is an organic glass, made from a thermosetting resin. The polymer matrix of said material is obtained by polymerization of a polymerizable composition comprising at least one polythiol and at least one polymerizable compound of formula (I): 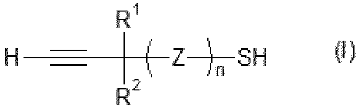 in which R¹ and R² represent, independently of each other, a hydrogen atom, a substituted or unsubstituted alkyl group, an aryl group, or R¹ and R², taken together, form a divalent group of formula -R¹-R²-, in which -R¹-R²- represents a substituted or unsubstituted alkylene group,

Z represents a divalent group, connected to the adjacent mercapto -SH group through a carbon atom, and n = 0 or 1.

The optical material of the invention can be used as the substrate of an optical article, preferably an optical lens or lens blank, more preferably an ophthalmic lens or lens blank, such as a plastic eyeglass lens. It can also be used as a coating.

The term "ophthalmic lens" is used to mean a lens adapted to a spectacle frame to protect the eye and/or correct the sight. Said lens can be chosen from afocal, unifocal, bifocal, trifocal, progressive, plano, solar and Fresnel lenses or any other kind of lenses having a discontinuous surface.

Although ophthalmic optics is a preferred field of the invention, it will be understood that this invention can be applied to optical articles of other types, such as, for example, lenses for optical instruments, in photography or astronomy, optical sighting lenses, ocular visors, optics of lighting systems, screens, glazing, windshields, sport masks, face shields, goggles, optical coatings or adhesives, etc.

If the optical article is an optical lens, it may be coated on its front main surface, rear main side, or both sides with one or more functional coatings. As used herein, the rear face of the substrate is intended to mean the face which, when using the article, is the nearest from the wearer's eye. It is generally a concave face. On the contrary, the front face of the substrate is the face which, when using the article, is the most distant from the wearer's eye. It is generally a convex face. The optical article can also be a plano article.

A substrate, in the sense of the present invention, should be understood to mean an uncoated substrate, and generally has two main faces. The substrate may in particular be made of the present optical material having the shape of an optical article, for example an ophthalmic lens destined to be mounted in glasses. In this context, the term "substrate" is understood to mean the base constituent material of the optical article and more particularly of the optical lens. This material may act as support for a stack of one or more coatings or layers.

From the viewpoint of reducing the thickness of a lens, a plastic material having a high refractive index is desired. The refractive index of the optical material according to the invention is higher than or equal to 1.50, preferably 1.52 or greater, more preferably 1.54 or greater, more preferably 1.56 or greater, more preferably 1.58 or greater, more preferably 1.60 or greater, and still more preferably 1.65 or greater, 1.67 or greater, 1.70 of greater, 1.72 of greater, or 1.74 or greater, and it is preferably 1.80 or less, more preferably 1.75 or less. Unless otherwise specified, the refractive indexes referred to in the present application are expressed at 25°C at a wavelength of 550 nm.

The refractive index of the optical material can be tuned by adapting the structure of the polymerizable precursors, in particular the weight amount represented by sulfur atoms in the monomers. The refractive index of the material can also be increased by the presence of one or more aromatic groups in the structure of at least one polymerizable precursor.

In one embodiment, the optical material according to the invention is thin, i.e., it preferably has a center thickness of 2 mm or less, more preferably 1.5 mm or less and even better 1.2 or 1.1 mm or less.

The optical material according to the invention preferably has a glass transition temperature higher than or equal to 70, 75, 80 or 85°C. It is preferably lower than or equal to 200°C. The glass transition temperature can be measured by DMA (dynamic mechanical analysis). The glass transition temperature of the present optical material is advantageously higher than that of most optical materials obtained by thiol-ene reactions due to a higher crosslinking density provided by the present polymerizable compounds.

The modulus of elasticity E (or Young's modulus, or storage modulus, or tensile modulus of elasticity) of the optical material according to the invention is preferably higher than or equal to 2.5, 3 or 3.5 GPa. The modulus of elasticity E of a material evaluates the ability of the material to deform under the effect of a force applied. It can be measured by DMA (dynamic mechanical analysis).

The sulfur content of the optical material according to the invention preferably ranges from 20 to 65 % by weight, relative to the optical material total weight. In one embodiment, said sulfur weight content of the optical material ranges from 30 to 65 % or 35 to 55 %. It can be adapted by changing the nature of the polymerizable precursors.

The optical material according to the invention preferably has a relative light transmission factor in the visible spectrum Tv higher than or equal to 70 %, preferably higher than or equal to 75 %, more preferably higher than or equal to 80 %, and better higher than or equal to 85 %.

The Tv factor, also called "luminous transmission" of the system, is such as defined in ISO standard 13666:1998 and is measured according to the standard ISO 8980-3. It is defined as the average transmission in the 380-780 nm wavelength range that is weighted according to the sensitivity of the eye at each wavelength of the range and measured under D65 illumination conditions (daylight). Transmissions are expressed for 2 mm thick optical articles, measured at the center of the optical article and at a normal incidence of the light beam (0° from the normal).

The polymerizable composition leading to the optical material is composed of two main components, namely a polythiol and a terminal alkyne thiol of formula (I), which contains at least two different reactive (polymerizable) functions, namely at least one mercapto -SH group, and at least one terminal alkyne -C=CH group. The monomers of the polymerizable composition react to form, in particular, thioether bonds.

The monomer compound of formula (I) is generally a heterodifunctional compound, but may in fact comprise more than two kinds of reactive functions, e.g., when at least one of its substituents (R¹, R² Z) comprises another kind of reactive function.

In one embodiment, said compound of formula (I) and said polythiol are different compounds.

The polymerizable composition can comprise only one type of compound of formula (I), or a mixture of compounds of formula (I) having different structures.

The polymerizable composition can comprise additional polymerizable compounds that are neither compounds of formula (I) according to the invention nor polythiols. In one embodiment, such additional polymerizable compounds (comonomers) are copolymerizable with either compounds of formula (I) according to the invention or polythiols, or both.

The polythiols and compounds of formula (I) according to the invention preferably represent at least 50 % by weight relative to the total weight of polymerizable compounds present in said polymerizable composition, more preferably at least 60 %, 70 %, 80 %, 90 %, 95 %, 99 %, or 100 % by weight relative to the total weight of polymerizable compounds present in said polymerizable composition.

Examples of polymerizable compounds that are neither compounds of formula (I) according to the invention nor polythiols include polyols, polyamines, polyisocyanates, polyisothiocyanates, epoxy-containing compounds.

In one embodiment, the polymerizable composition comprises from 30 % to 80 % by weight of compounds of formula (I) relative to the total weight of polymerizable compounds present in said polymerizable composition, more preferably from 35 % to 75 % by weight.

In one embodiment, the polymerizable composition comprises from 20 % to 75 % by weight of polythiols relative to the total weight of polymerizable compounds present in said polymerizable composition, more preferably from 25 % to 70 % by weight.

Generally, the polymerizable composition contains polymerizable compounds in amounts adapted so that the molar ratio of SH groups to alkyne groups present in said polymerizable compounds is from 1.6 to 2.4, more preferably from 1.8 to 2.2, ideally 2. Those polymerizable compounds are compounds of formula (I), which may be identical or different, polythiols, and optional polymerizable compounds of other categories. Indeed, a stoichiometric reaction between polythiols and compounds of formula (I) requires the use of two equivalents of thiol functions for one equivalent of alkyne functions in the polymerizable composition, when no other functions are present.

The compound of formula (I) is defined as a compound comprising at least one sulfhydryl (mercapto) group. It preferably comprises 1, 2, 3 or 4 mercapto groups, more preferably 1.

The compound of formula (I) is defined as a compound comprising at least one alkyne bond. It preferably comprises one or two alkyne bonds, more preferably one.

In one embodiment, the compound of formula (I) is such that the ratio of thiol groups / alkyne groups per molecule of compound of formula (I) is equal to 1.

In one embodiment, the compound of formula (I) comprises at least one thioether function, preferably one. In another embodiment, the compound of formula (I) comprises at least one ester function (C(O)O), preferably one.

In the present application, the term "alkyl" denotes a linear or branched, cyclic or acyclic, saturated or unsaturated hydrocarbon-based radical connected to the rest of the molecule via an sp³ carbon atom, containing preferably from 1 to 25 carbon atoms, especially including acyclic groups containing from 1 to 8 carbon atoms such as methyl, ethyl, n-propyl, isopropyl, n-butyl and n-hexyl groups, cycloalkyl groups preferably containing from 3 to 7 carbon atoms, cycloalkylmethyl groups preferably containing from 4 to 8 carbon atoms.

By "substituted alkyl" group is understood an alkyl group as defined above, connected to the rest of the molecule via an sp³ carbon atom and in which one or more methylene hydrogen atoms are replaced with a substituent. The substituted alkyl group can be substituted by one or a plurality of aryl groups and/or one or a plurality of heteroatoms such as N, S, O or a halogen atom (fluorine, chlorine, bromine or iodine). Mention will be made by way of examples of arylalkyl groups such as the trityl group (-CPh₃), the benzyl group or the 4-methoxybenzyl group, alkoxyalkyl groups, particularly dialkoxymethyl groups such as the diethoxymethyl or dimethoxymethyl groups, the groups CH₂CO₂R¹¹, wherein R¹¹ represents an optionally substituted alkyl or aryl group.

The term "heteroalkyl" denotes an alkyl group as defined above, connected to the rest of the molecule via an sp³ carbon atom, wherein one or a plurality of carbon atoms of the alkyl chain have been replaced with a heteroatom such as nitrogen (e.g., NH, N-alkyl...), oxygen, phosphorus, or sulfur (e.g., SO, SO₂...).

The term "aryl" denotes an aromatic monovalent carbocyclic radical, connected by an sp² carbon atom, including a single ring (for example a phenyl group) or multiple condensed rings (for example the naphthyl, terphenyl groups), which may optionally be substituted by one or a plurality of groups such as, without limitation, alkyl (for example methyl), hydroxyalkyl, aminoalkyl, hydroxyl, thiol, amino, halogeno (fluoro, bromo, iodo, chloro), nitro, alkylthio, alkoxy (for example methoxy), aryloxy, mono-alkylamino, dialkylamino, acyl, carboxyl, alkoxycarbonyl, aryloxycarbonyl, hydroxysulphonyl, alkoxysulphonyl, aryloxysulphonyl, alkylsulphonyl, alkylsulphinyl, cyano, trifluoromethyl, tetrazolyl, carbamoyl, alkylcarbamoyl, dialkylcarbamoyl groups. Alternatively, two adjacent positions of the aromatic ring may be substituted by a methylenedioxyl or ethylenedioxyl group. The aryl group preferably comprises from 6 to 15 carbon atoms.

The term "heteroaryl" denotes an aryl group as defined above, connected to the rest of the molecule via an sp² carbon atom, wherein one or a plurality of carbon atoms of the aromatic ring(s) have been replaced with a heteroatom such as nitrogen, oxygen, phosphorus, or sulfur. The heteroaryl groups may be structures with a single or a plurality of aromatic rings, or structures with a single or a plurality of aromatic rings coupled with one or a plurality of non-aromatic rings. In the structures having a plurality of rings, the rings may be fused, bonded covalently or bonded together via a divalent common group such as a methylene, ethylene, carbonyl group. Examples of heteroaryl groups are the thiophene (2-thienyl, 3-thienyl), pyridine (2-pyridyl, 3-pyridyl, 4-pyridyl), isoxazole, phthalimide, pyrazole, indole, furan groups and the benzofused analogues thereof, phenyl pyridyl ketone, quinoline, phenothiazine, carbazole, benzopyranone.

The suffix "-ene" is used to describe a divalent group. Thus, any of the monovalent groups defined herein can be modified with the suffix "-ene" to describe a divalent version of that moiety. For example, a divalent aryl group is "arylene", a divalent alkyl group is "alkylene". Alkylene groups are connected to the rest of the molecule via two sp³ carbon atoms. Arylene groups are connected to the rest of the molecule via two sp² carbon atoms.

A (hetero)alkylene group represents a heteroalkylene or alkylene group. A (hetero)arylene group represents a heteroarylene or arylene group.

Examples of alkylene groups include linear C1-C10 alkylene groups, for example a methylene group -CH₂-, an ethylene group -CH₂-CH₂-, 1,3-propylene, a butylene or a hexylene group, especially 1,4-butylene and 1,6-hexylene and branched C3-C10 alkylene radicals such as 1,4-(4-methyl pentylene), 1,6-(2,2,4-trimethyl hexylene), 1,5-(5-methyl hexylene), 1,6-(6-methyl heptylene), 1,5-(2,2,5-trimethyl hexylene), 1,7-(3,7-dimethyl octylene), 2,2-(dimethylpropylene), 1,5-pentylene, 1,1-dimethylpentylene and 1,6-(2,4,4-trimethyl hexylene) radicals. Preferred cycloalkylene radicals include cyclopentylene and cyclohexylene radicals, optionally substituted especially by alkyl groups.

Non-limiting examples of heteroalkylene groups include -CH₂SCH₂-, -CH₂CH₂SCH₂CH₂-, -CH₂OCH₂-, -CH₂CH₂OCH₂CH₂-, -CH₂-CH₂-S-CH₂-CH(CH₂SH)-S-CH₂-CH₂-.

Examples of arylene groups include 2,4-tolylene, 2,6-tolylene, 2,4-naphthylene, 2,6-naphthylene, 1,5-naphthylene, 1,4-phenylene, 1,4-bisphenylene (-*p*-C₆H₄-p-C₆H₄-), 2-methyl-1,3-phenylene, 4-methyl-1,3-phenylene, tetramethylxylylene, 1,4-phenylene-methylene-1,4-phenylene (4,4-biphenylenemethylene).

In the present application, (hetero)alkylene and/or alkyl groups preferably comprise from 1 to 5 carbon atoms, more preferably from 1 to 4 carbon atoms, from 1 to 3 carbon atoms, or from 1 to 2 carbon atoms. It is preferable to have short substituent chains, in particular short alkyl and/or alkylene chains, since long chains tend to decrease the refractive index of the resulting optical material.

In one embodiment, R¹ = R².

The group -R¹-R²- defined in the present application preferably represents a linear alkylene group such as -(CH₂)₅- (thus forming a cyclohexyl group with the carbon atom connected to the alkyne bond) or -(CH₂)₄-.

The R¹ and R² groups preferably represent, independently of each other, a hydrogen atom or a substituted or unsubstituted alkyl group, the alkyl group being preferably a C1-C5 alkyl group, more preferably a C1-C2 alkyl group and ideally a methyl group.

In preferred embodiments of the invention, R¹ = R² = H, or R¹ = R² = CH₃, or R¹ = H and R² = CH₃.

In another embodiment, Z is selected from substituted or unsubstituted:
- (hetero)alkylene groups, (hetero)arylene groups,
- carbonyl(hetero)alkylene groups, carbonyl(hetero)arylene groups,
- oxycarbonyl(hetero)alkylene groups, oxycarbonyl(hetero)arylene groups,
- carbonyloxy(hetero)alkylene groups, carbonyloxy(hetero)arylene groups,
- oxy(hetero)alkylene groups, oxy(hetero)arylene groups,
- thio(hetero)alkylene groups, thio(hetero)arylene groups,
- sulfo(hetero)alkylene groups, sulfo(hetero)arylene groups,
- amino(hetero)alkylene groups, amino(hetero)arylene groups,
- alkylamino(hetero)alkylene groups, alkylamino(hereto)arylene groups,
- arylamino(hetero)alkylene groups, arylamino(hetero)arylene groups,
- aminocarbonyl(hetero)alkylene groups, aminocarbonyl(hetero)arylene groups,
- alkylaminocarbonyl(hetero)alkylene groups, alkylaminocarbonyl(hereto)arylene groups,
- arylaminocarbonyl(hetero)alkylene groups, arylaminocarbonyl(hetero)arylene groups,
- carbonylamino(hetero)alkylene groups, carbonylamino(hetero)arylene groups,
- carbonylalkylamino(hetero)alkylene groups, carbonylalkylamino(hereto)arylene groups,
- carbonylarylamino(hetero)alkylene groups, carbonylarylamino(hetero)arylene groups, and combinations of the above divalent groups.

The carbonyl function is -C(O)-, the oxycarbonyl function is -O-C(O)-, the carbonyloxy function is -C(O)O-, the sulfo function is -SO₂-. An oxycarbonylalkylene group is -O-C(O)-alkylene, while a carbonyloxyalkylene group is -C(O)O-alkylene, etc.

Combinations of the above divalent groups include, without limitation, combinations of groups of the same or a different category, for example cycloalkylene-alkylene groups, biscycloalkylene groups, biscycloalkylene-alkylene groups, arylene-alkylene groups (such as the benzylene group), biarylene groups such as bisphenylene, biarylene-alkylene groups, arylene-oxyalkylene groups, poly[oxyalkylene] groups, poly[oxy(hetero)arylene] groups, poly[thioalkylene] groups, poly[thio(hetero)arylene] groups.

Specific examples of Z groups include, apart from the alkylene and arylene groups mentioned above, -S-CH₂-, -S-CH₂-CH₂-, -S-CH₂-CH₂-S-CH₂-CH₂-, -CH₂SCH₂-, -S-CH₂-CH₂-S-CH₂-CH(CH₂SH)-S-CH₂-CH₂-, -O-C(O)-CH₂-, -O-C(O)-CH₂-CH₂-, -O-C(O)-CH(CH₃)-, - CONH(CH₂)₃-, -OCH₂CH(OH)CH₂-, polyoxyethylene groups such as -(OCH₂CH₂)ₙ- with n being an integer ranging from 2 to 10, polyoxypropylene groups.

Other useful divalent Z groups comprise (hetero)alkylene groups or (hetero)arylene groups connected to any one of the following divalent groups: -OC(O)O-, -C(O)C(O)-, - OC(O)C(O)O-, -C(O)OC(O)-, -C(S)-.

In one embodiment, the groups Z comprise an alkenylene or alkynylene group.

The most preferred Z groups are thio(hetero)arylene groups, thio(hetero)alkylene groups, and oxycarbonyl(hetero)alkylene groups, the (hetero)alkylene and (hetero)arylene groups being substituted or unsubstituted.

In one embodiment, said polymerizable compound is selected from the compounds of formula (I): 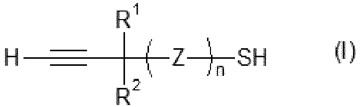 in which R¹ and R² are as defined above and preferably represent, independently of each other, a hydrogen atom or a substituted or unsubstituted alkyl group, Z is as defined above and preferably represents a thio(hetero)arylene group, a thio(hetero)alkylene group, or a poly[thio(hetero)alkylene] group, the (hetero)alkylene and (hetero)arylene groups being substituted or unsubstituted, and n = 0 or 1.

Specific examples of such compounds of formula (I) in which n = 0 are shown hereunder:

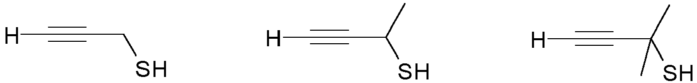

A useful category of compounds of formula (I) in which n = 1 comprises thioether compounds of formula (III): 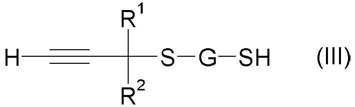 in which R¹ and R² are as defined above and preferably represent, independently of each other, a hydrogen atom or a substituted or unsubstituted alkyl group, G represents a divalent group connected to the adjacent mercapto -SH group through a carbon atom, and preferably connected on both sides through a carbon atom.

The G groups are preferably chosen from substituted or unsubstituted (hetero)alkylene groups, substituted or unsubstituted (hetero)arylene groups. G is preferably selected from, substituted or unsubstituted, alkylene, typically a C2-C4 alkylene group, arylene, heteroarylene, or heteroalkylene such as an alkylene-thio-alkylene group like the group -(CH₂)₂-S-(CH₂)₂-.

Specific examples of such compounds of formula (III) are shown hereunder:

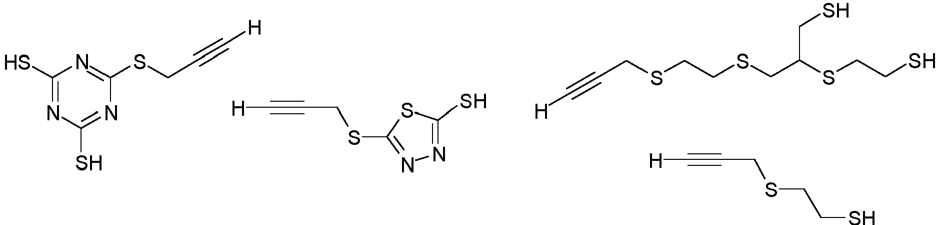

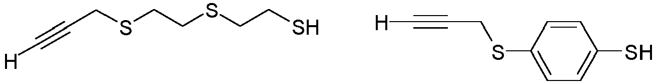

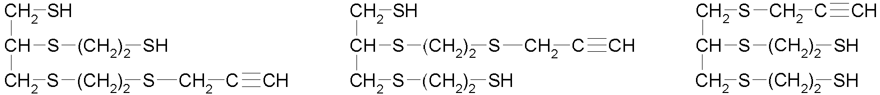

Another useful category of polymerizable compounds according to the invention comprises compounds of formula (II) having an ester function (C(O)O): 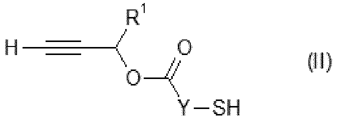 in which R¹ is as defined above and preferably represents a hydrogen atom, a substituted or unsubstituted alkyl group, or an aryl group, Y represents an (hetero)alkylene group.

Specific examples of such compounds are mercaptocarboxylic acid esters such as the compounds shown hereunder:

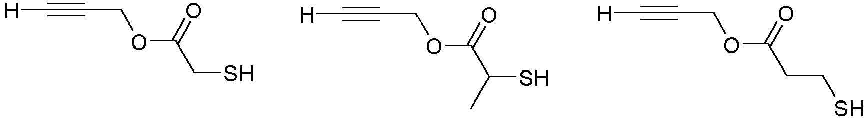

The polymerizable compounds of general formula (I), (II) and (III) can be easily synthesized from widely available and relatively cheap raw materials, such as propargyl alcohol, propargyl chloride, 1-ethynyl-1-cyclohexanol, 3-butyne-2-ol, 2-methyl-3-butyn-2-ol, ethane dithiol, thiolactic acid, mercaptopropionic acid, thioglycolic acid, etc., through chemical reactions well known to those skilled in the art. The widespread use of these raw materials used to synthesize the monomers of the present invention enables very competitive cost in regard of the refractive index achieved.

The terminal alkyne thiol compounds according to the invention can be stored in the presence of an antioxidant such as BHT (2,6-bis(1,1-dimethylethyl)-4-methylphenol) to prevent addition of thiol groups on the alkyne bond.

The polythiols that may be used in the present invention are defined as compounds comprising at least two sulfhydryl (mercapto) groups, in other words dithiols, trithiols, tetrathiols etc. Polythiols pre-polymers or oligomers may be used. The polythiol may be any suitable polythiol having two or more, preferably two, three or four thiol functions.

By pre-polymer, it is meant a polymer or oligomer comprising pre-polymer molecules. By pre-polymer molecule, it is meant a macromolecule or oligomer molecule capable of entering, through reactive (polymerizable) groups, into further polymerization, thereby contributing more than one monomeric unit to at least one chain of the final macromolecule. It is generally formed from two or more different monomers.

In one embodiment of the invention, said polythiol is a compound of formula:

R³(SH)ₙ₁ (IV)

wherein n1 represents an integer ranging from 2 to 6 and R³ represents an aliphatic, alicyclic, heterocyclic or aromatic group.

The preferred polythiol monomers and/or oligomers suitable in accordance with the present invention, there may be cited aliphatic polythiols such as trimethylolpropanetris(2-mercaptoacetate), trimethylolpropanetris(3-mercaptopropionate), trimethylolethanetris(2-mercaptoacetate), trimethylolethanetris(3-mercaptopropionate), pentaerythritol tetrakis(2-mercaptoacetate), pentaerythritol tetrakis(3-mercaptopropionate), dipentaerythritol hexakis(3-mercaptopropionate), bis(mercaptomethyl)sulfide, bis(mercaptomethyl)disulfide, bis(mercaptoethyl)sulfide, bis(mercaptoethyl)disulfide, bis(mercaptopropyl)sulfide, bis(mercaptopropyl)disulfide, 2,3-bis((2-mercaptoethyl)thio)-1-propanethiol of formula (VI), 4,8-dimercaptomethyl-1,11-dimercapto-3,6,9-trithiaundecane of formula (VII), 4,7-dimercaptomethyl-1,11-dimercapto-3,6,9-trithiaundecane, 5,7-dimercaptomethyl-1,11-dimercapto-3,6,9-trithiaundecane, 2,5-bis (mercaptomethyl)-1,4-dithiane, and 2,5-bis[(2-mercaptoethyl)thiomethyl]-1,4-dithiane, 1-(1'-mercaptoethylthio)-2,3-dimercaptopropane, 1-(2'-mercapropylthio)-2,3-dimercaptopropane, 1-(3'-mercapropylthio)-2,3-dimercaptopropane, 1-(4'-mercabutylthio)-2,3-dimercaptopropane, 1-(5'-mercapentylthio)-2,3-dimercaptopropane, 1-(6'-mercahexylthio)-2,3-dimercaptopropane, 1,2-bis-(4'-mercaptobutylthio)-3-mercaptopropane, 1,2-bis-(5'-mercaptopentylthio)-3- mercaptopropane, 1,2-bis-(6'-mercaptohexylthio)-3-mercaptopropane, 1,2,3-tris(mercaptomethylthio)propane, 1,2,3-tris-(3'-mercaptopropylthio)propane, 1,2,3-tris-(2'-mercaptoethylthio)propane, 1,2,3-tris-(4'-mercaptobutylthio)propane, 1,2,3-tris-(6'-mercaptohexylthio)propane, methanedithiol, 1,2-ethanedithiol, 1,1-propanedithiol, 1,2-propanedithiol, 1,3-propanedithiol, 2,2-propanedithiol, 1,6-hexanethiol-1,2,3-propanetrithiol, 1,2-bis(2'-mercaptoethylthio)-3-mercaptopropane, tris[2-(3-mercaptopropionyloxy)ethyl] isocyanurate of formula (VIII), 2-mercaptoethyl 2-mercaptoacetate of formula (IX), 2-mercaptoethyl 3-mercaptopropionate, 1,1,3,3-tetrakis (mercaptomethylthio) propane, 4,6-bis (mercaptomethylthio)-1,3-dithiane, 1,1,2,2-tetrakis (mercaptomethylthio) ethane, 3-mercaptomethyl-1,5-dimercapto-2,4-dithiapentane, tris (mercaptomethylthio) methane, ethylene glycol bis (3-mercaptopropionate), butylene glycol bis (3-mercaptopropionate), 2-(2,2-bis (mercaptomethylthio) ethyl)-1,3-dithietane,

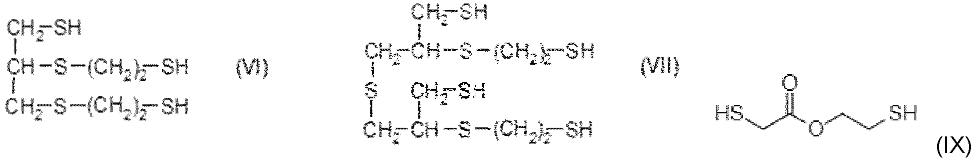

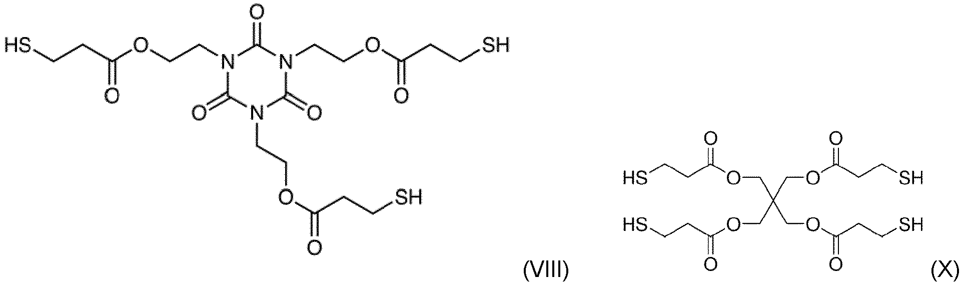

Further examples of polythiols are shown in the formulae below or can be found in WO 2014/133111, EP 394495, US 4775733 or EP 1877839:

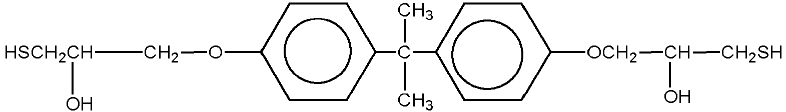

C₂H₅C(CH₂COOCH₂CH₂SH)₃

In one embodiment of the invention, said polythiol is selected from the group consisting of pentaerythritol tetrakis(3-mercaptopropionate), pentaerythritol tetrakis(2-mercaptoacetate), tris(3-mercaptopropionate) trimethylolpropane, tris(2-mercaptoacetate) trimethylolpropane, 2,3-bis((2-mercaptoethyl)thio)-1-propanethiol, ethylene glycol bis (3-mercaptopropionate), butylene glycol bis (3-mercaptopropionate), 2-mercaptoethyl 2-mercaptoacetate, 2-mercaptoethyl 3-mercaptopropionate, 5,7-dimercaptomethyl-1,11-dimercapto-3,6,9-trithiaundecane, 4,7-dimercaptomethyl-1,11-dimercapto-3,6,9-trithiaundecane, 4,8-dimercaptomethyl-1,11-dimercapto-3,6,9-trithiaundecane, 2,5-bis (mercaptomethyl)-1,4-dithiane, bis (mercaptoethyl) sulfide, 1,1,3,3-tetrakis (mercaptomethylthio) propane, 4,6-bis (mercaptomethylthio)-1,3-dithiane, 2-(2,2-bis (mercaptomethylthio) ethyl)-1,3-dithietane, 1,1,2,2-tetrakis (mercaptomethylthio) ethane, 3-mercaptomethyl-1,5-dimercapto-2,4-dithiapentane, tris (mercaptomethylthio) methane, dipentaerythritol hexakis(3-mercaptopropionate), tris[2-(3-mercaptopropionyloxy)ethyl] isocyanurate, and ethanedithiol.

The most preferred polythiols are 2-mercaptoethyl 2-mercaptoacetate of formula (IX), pentaerythritol tetrakis (3-mercaptopropionate) of formula (X) and 2,3-bis((2-mercaptoethyl)thio)-1-propanethiol of formula (VI).

The polymerizable composition according to the invention may also include additives which are conventionally employed in polymerizable compositions intended for molding optical articles, in particular ophthalmic lenses, in conventional proportions, namely catalysts/polymerization initiators, photochromic agents, UV absorbers, perfumes, deodorants, resin modifiers, color balancing agents, chain extenders, crosslinking agents, free radical scavengers such as antioxidants or hindered amine light stabilizers (HALS), dyes, pigments, fillers, adhesion accelerators, inhibitors, anti-yellowing agents and mold release agents.

UV absorbers are frequently incorporated into optical materials in order to reduce or prevent UV light from reaching the retina (in particular in ophthalmic lens materials). The UV absorber that may be used in the present invention preferably have the ability to at least partially block light having a wavelength shorter than 400 nm, but can also have an absorption spectrum extending to the visible blue light range of the electromagnetic spectrum (400-450 nm), in particular 420-450 nm.

Said UV absorbers both protect the user's eye from UV light and the optical material itself, thus preventing it from weathering and becoming brittle and/or yellow. The UV absorber according to the invention can be, without limitation, a benzophenone-based compound, a benzotriazole-based compound or a dibenzoylmethane-based compound, preferably a benzotriazole compound. Suitable UV absorbers include without limitation 2-(2-hydroxyphenyl)-benzotriazoles such as 2-(2-hydroxy-3-tert-butyl-5-methylphenyl)-5-chlorobenzotriazole (Seesorb^{®} 703 / Tinuvin^{®} 326), or other allyl hydroxymethylphenyl chlorobenzotriazoles, 2-(5-chloro-2H-benzotriazol-2-yl)-6-(1,1-dimethylethyl)-4-methylphenol (Viosorb^{®} 550), n-octyl-3-[3-tert-butyl-4-hydroxy-5-(5-chloro-2H-benzotriazol-2-yl)phenyl] propionate (Eversorb^{®} 109), 2-(2-hydroxy-5-methoxyphenyl)benzotriazole, 2-(2-hydroxy-5-butoxyphenyl)benzotriazole and also Tinuvin^{®} CarboProtect^{®} from BASF. Preferred absorbers are of the benzotriazole family. Other examples of benzotriazole UV absorbers protecting from blue light can be found in WO 2017/137372.

The amount of UV absorber compounds according to the invention used herein is an amount sufficient to provide a satisfactory protection from UV light but not excessive so as to prevent precipitation. The UV absorber compounds are generally present in an amount ranging from 0.05 to 4 % by weight relative to the optical material total weight (or per 100 parts by weight of the polymerizable compounds present in the composition or relative to the weight of the optical material composition), preferably from 0.1 to 3 % by weight, more preferably from 0.1 to 2 % by weight.

Among the release agents that may be used in the invention, there may be cited mono and dialkyl phosphates, alkyl ester phosphates, silicones, fluorinated hydrocarbon, fatty acids and ammonium salts. The preferred release agents are mono and dialkyl phosphates, alkyl ester phosphates and mixtures thereof. Such release agents are disclosed inter alia in US 4975328 and EP 271839. The release agent is preferably used in an amount lower than or equal to 1 % by weight based on the total weight of the polymerizable compounds present in the polymerizable composition.

The optical material composition is photopolymerizable and can contain at least one system for initiating the polymerization (initiator), preferably a photoinitiator. A photoinitiator represents a molecule that absorbs light and generates reactive species (ions or radicals) that initiate a chemical reaction or transformation.

Photoinitiators can be selected for example from haloalkylated aromatic ketones such as chloromethylbenzophenones, benzoin and benzoin alkyl ethers such as benzoin methyl ether, benzoin ethyl ether, benzoin isopropyl ether, benzoin isobutyl ether, benzoin, dialkoxyacetophenones such as diethoxyacetophenone and 2,2-dimethoxy-2-phenylacetophenone, benzylideneacetophenone, hydroxy ketones such as (1-[4-(2-hydroxyethoxy)-phenyl]-2-hydroxy-2-methyl-1-propan-1-one) (Irgacure^{®} 2959 from CIBA), 2,2-di-sec-butoxyacethophenone, 2,2-diethoxy-2-phenyl-acetophenone, 1-hydroxy-cyclohexyl-phenyl-ketone (Irgacure^{®} 184 from CIBA) and 2-hydroxy-2-methyl-1-phenylpropan-1-one (such as Darocur^{®} 1173 sold by CIBA), alpha amino ketones, particularly those containing a benzoyl moiety, otherwise called alpha-amino acetophenones, for example 2-methyl 1-[4-phenyl]-2-morpholinopropan-1-one (Irgacure^{®} 907 from CIBA), (2-benzyl-2-dimethyl amino-1-(4-morpholinophenyl)-butan-1-one (Irgacure^{®} 369 from CIBA), monoacyl and bisacyl phosphine oxides and sulphides, such as phenylbis(2,4,6-trimethylbenzoyl)-phosphine oxide (Irgacure^{®} 819 sold by CIBA, and Irgacure^{®} 2022, which is a blend containing Irgacure^{®} 819 and Darocur^{®} 1173), 2,4,6,-trimethylbenzoylethoxydiphenyl phosphine oxide, triacyl phosphine oxides, 2,2'-bis(2-chlorophenyl)-4,4',5,5'-tetraphenyl-1,2'-biimidazole and mixtures thereof.

Free radical initiators such as peroxides and azo compounds, either thermally or photo-catalyzed, can also be used, as well as Lewis acids, such as triarylsulfonium hexafluoroantimonate salts or diaryliodonium salts.

Initiators shall be used in the polymerizable composition in an amount sufficient to promote the polymerization reaction. They are generally present in an amount ranging from 0.05 to 10 % by weight, preferably from 0.2 % to 5 %, and more preferably from 0.25 % to 2 % by weight, relative to the total weight of polymerizable compounds present in the polymerization composition.

The polymerizable composition of the present invention can comprise a solvent for promoting the dissolution of additives. Any polar organic solvent can be used such as acetonitrile, tetrahydrofuran, dioxane, ethanol, 2-mercaptoethanol, acetone, or 3-methyl-2-butene-1-ol. The amount of solvent is generally kept below 2% by weight, based on the total weight of the polymerizable compounds present in the composition and preferably from 0 to 0.5% by weight, to avoid haze and bubbling.

The invention also relates to a process for the preparation of an optical material as described above, comprising polymerization of the polymerizable composition in the presence of at least one initiator, preferably a photoinitiator, followed by a thermal post-cure. The process preferably involves casting polymerization.

The mixing of the different constituents of the polymerizable composition can be performed by any known mixing technique such as those mentioned in US 5973098, preferably by introducing the constituents in a small reactor chamber and then mixing with a screw mixer.

A molding cavity of a casting mold assembly having any desirable shape can then be filled with the mixture of reactants.

The casting mold assembly generally comprises two mold parts defining two molding surfaces that cooperate to form a molding cavity when moved from an open position to a closed position. Each of the molding surfaces can be concave, convex, or planar, depending on the desired article shape. The molding surface can be convex, e.g., to form a concave substrate surface, or concave, e.g., to form a convex substrate surface.

More specifically, the optical material composition can be poured into the cavity of two mold parts held together using an annular closure such as a gasket or an adhesive tape.

An annular closure member can be disposed around the periphery of the two mold pieces and attached to them. The conventional way to fill such a two-piece mold is by causing the (liquid) optical material composition to flow into the molding cavity through a casting opening provided for this purpose in the closure member. In at least a partly automated process, the molding cavity to be filled is vertically aligned with a filling device that is adapted to deliver a particular quantity of molding material through a nozzle.

Depending on the desired characteristics of the resulting optical material, degassing can be performed under reduced pressure and/or filtration can be performed under increased pressure or reduced pressure before pouring the optical material composition in the mold assembly.

After pouring the composition in the casting mold assembly, preferably a lens casting mold assembly, polymerization, forming thioether bonds, preferably photopolymerization of the polymerizable composition is generally performed by irradiating the composition, preferably with ultraviolet light. Preferably, UV light wavelength ranges from 320 to 390 nm. UV light intensity typically ranges from 40 to 90 mW/cm² and the total exposure time to UV light, either in one shot or several shots, preferably ranges from 200 to 1650 seconds, more preferably from 200 to 600 seconds.

The final thermal post-cure can be performed in an oven or a heating device immersed in water according to a predetermined temperature program to cure the resin in the mold assembly. The curing temperature generally ranges from 60°C to 140°C. The curing time is preferably lower than 5 hours, more preferably lower than 4, 3 or 2 hours. As used herein, curing refers to a chemical process of converting monomers or oligomers into a polymer of higher molar mass and then into a network.

The resin molded product may then be annealed if necessary, at a temperature preferably ranging from 100°C to 150°C.

Thereafter, the mold assembly is withdrawn from the heating source, the annular closure member is removed and the polymerized optical material can be recovered after disassembly of the mold parts.

The present process can be used to manufacture a finished lens, having both sides at the required geometries, or a semi-finished lens, having one face that still needs to be surfaced at the required geometry.

In some applications, it is preferred that the main surface of the optical material be coated with one or more functional coating(s) to improve the optical and/or mechanical properties. The term "coating" is understood to mean any layer, layer stack or film which may be in contact with the substrate and/or with another coating, for example a sol-gel coating or a coating made of an organic resin. A coating may be deposited or formed through various methods, including wet processing, gaseous processing, and film transfer. These functional coatings classically used in optics may be, without limitation, an impact-resistant and/or adhesion primer, an abrasion-resistant and/or scratch-resistant coating, an antireflection coating, a polarized coating, a photochromic coating, or an antistatic coating, or a stack made of two or more such coatings, especially an impact-resistant primer coating coated with an abrasion and/or scratch-resistant coating.

The invention also relates to a polymerizable compound selected from the compounds of formulae (XI) [2-((2-mercaptoethyl)thio)-3-((2-(prop-2-yn-1-ylthio)ethyl)thio)-1-propanethiol], (XII) [2-((2-(prop-2-yn-1-ylthio)ethyl)thio)-3-((2-mercaptoethyl)thio)-1-propanethiol], (XIII) and (XIV) [6-(prop-2-yn-1-ylthio)-1,3,5-triazine-2,4-dithiol]:

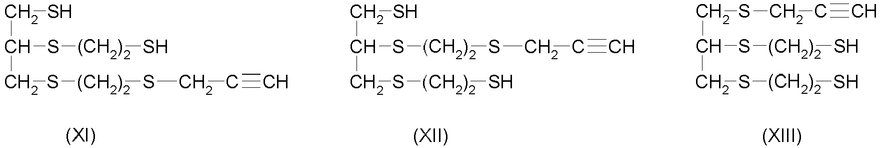

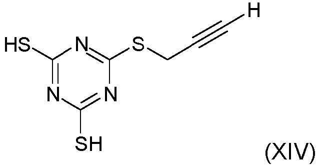

The compounds of formulae (XI), (XII) and (XIII) can be obtained by propargylation of 2,3-bis((2-mercaptoethyl)thio)-1-propanethiol.

The following examples illustrate the present invention in a more detailed, but non-limiting manner. Unless stated otherwise, all thicknesses disclosed in the present application relate to physical thicknesses. The percentages given in the tables are weight percentages.

### Examples

The polymerizable compounds can be selected, without limitation, from the compounds of formulae:

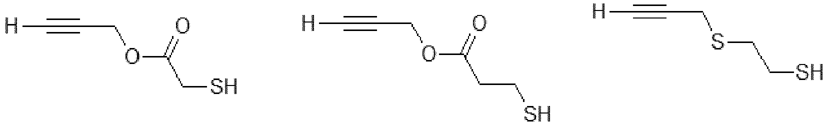

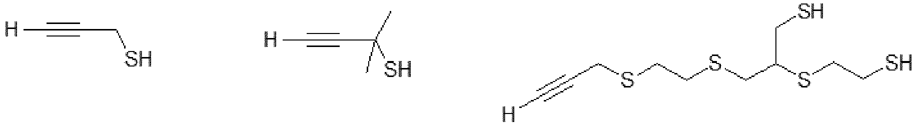

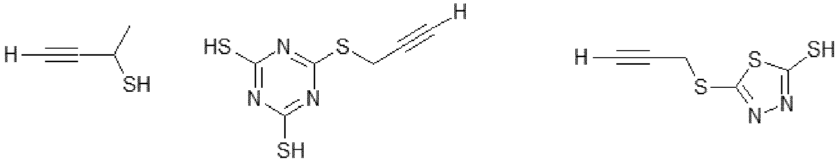

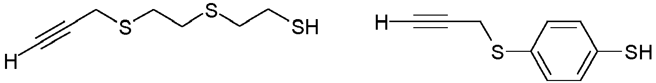

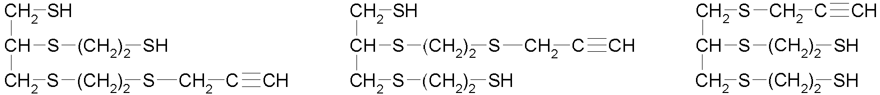

### 1. Chemicals used and polymerization conditions

Optical substrates were prepared by polymerization of equimolar amounts of a terminal alkyne thiol monomer and a polythiol monomer in the presence of a photoinitiator (2,2-dimethoxy-2-phenylacetophenone, 1 % by weight). The polymerizable composition also contained Zelec UN^{®} as a mold release agent.

The following terminal alkyne thiol monomers were used: Propargyl thioglycolate (examples 1-3), propargyl thiol (examples 4-6), 3-butyne-2-thiol (examples 7-9), 2-methyl-3-butyn-2-thiol (examples 10-12), 2-(2-propynylthio)ethanethiol (examples 13-15) and propargyl bismuthiol (examples 16-18).

Prop-2-yn-1-yl 3-mercaptopropanoate (compound of formula (II) in which Y = CH₂CH₂ and R¹ = H) can also be used (data not shown).

The following polythiol monomers were used: 2,3-bis((2-mercaptoethyl)thio)-1-propanethiol (CAS No. 131538-00-6), 2-mercaptoethyl 2-mercaptoacetate (CAS No. 38705-47-4) or pentaerythritol tetrakis (3-mercaptopropionate) (CAS No. 7575-23-7).

In a Duran bottle with a magnetic stirrer, the terminal alkyne thiol monomer of formula (I) and the polythiol monomer were mixed, followed by the photoinitiator and the mold release agent. The composition was stirred at room temperature for 30 minutes and allowed to degas for 10 minutes to avoid bubbles in the final material.

Cleaned convex and concave plano glass molds of high refractive index having 65-80 mm diameter were assembled with a tape. A center thickness adjustment was made to obtain 2 mm thick samples.

The assembled molds were filled with the above monomer formulation using a cleaned syringe, and the polymerization reaction was carried out by UV-irradiation for 4 minutes (365 nm), followed by a thermal post-cure (80°C, 1h) in an oven from ShenZhen Height-LED Technology Company Limited. The molds were then disassembled to obtain lenses comprising a body of a thermoset material.

### 2. Synthesis of terminal alkyne thiol monomers

### Preparation of propargyl thioglycolate (examples 1-3, also known as prop-2-yn-1-yl 2-mercaptoacetate)

Propargyl alcohol (0.1 mol, 5.605 g), BHT (2,6-bis(1,1-dimethylethyl)-4-methylphenol, 0.1 g), thioglycolic acid (0.105 mol, 9.673 g), p-toluene sulfonic acid (0.2 g) and 7 g of molecular sieves were introduced in a flask. The mixture was stirred at 60°C for 24h, then filtered and dissolved in dichloromethane. The organic phase was cleaned three times with NaHCO₃ 10 % and one time with deionized water, dried over sodium sulfate and the solvent evaporated to leave a pale yellow oil.

### Preparation of propargyl thiol (examples 4-6)

Potassium thioacetate (6.85 g, 0.06 mol) was introduced in a flask containing methanol (200 mL), followed by propargyl chloride (3.73 g, 0.05 mol), and the resulting mixture was further stirred for 2h. 7 mL of concentrated hydrochloric acid were added, and the solution was warmed to 50°C for 2h. The reaction mixture was then evapoated (rotavapor) and the residue taken up in 20 mL of methylene chloride, washed with 0.1 M hydrochloric acid and water. The organic phase was dried, and the solvent stripped under reduced pressure to leave a yellow oil.

### Preparation of 3-butyne-2-thiol (examples 7-9)

The product was obtained similarly as propargyl thiol, but starting from 3-bromo-1-butyne.

### Preparation of 2-methyl-3-butyn-2-thiol (examples 10-12)

The product was obtained similarly as propargyl thiol, but starting from 3-chloro-3-methyl-1-butyne.

### Preparation of propargyl bismuthiol (examples 16-18)

Propargyl chloride (14.8 g) in ethanol (50 g) was added with stirring to a mixture containing 2,5-dimercapto-1,3,4-thiadiazole (30 g), ethanol (400 g), NaOH (8 g), and water (40 g). The resulting mixture was refluxed for 2 hours, poured into ice (1500 parts by weight). The precipitate was filtered off and recrystallized from CHCl₃-petroleum ether to give 2-propinylthio-5-mercapto-1,3,4-thiadiazole (propargyl bismuthiol), having a melting point of 113-114°C.

2-(2-propynylthio)ethanethiol (examples 13-15) can be prepared similarly from propargyl chloride and ethane dithiol.

### 3. Testing methods

The refractive indexes of the polymers can be determined by ellipsometry at the wavelength of 550 nm in the manner disclosed in WO 2015/166144.

### 4. Optical articles prepared and characterizations

The tables hereunder indicate the nature of the monomers used, the refractive index of the resulting polymer and its theoretical sulfur content, by weight. SH functions were used in an amount balancing the amount of alkyne functions, i.e., the molar ratio of SH/alkyne groups present in the polymerizable compounds was equal to 2. The resulting new polymers had high refractive indexes, ranging from 1.60 to 1.72, depending on the structure of the starting monomer.

| Example | 1 | 2 | 3 |
|---|---|---|---|
| Alkyne thiol | 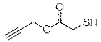 | 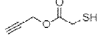 | 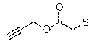 |
| Alkyne thiol amount | 63.1 % | 51.59 % | 59.99 % |
| Polythiol | 2-mercaptoethyl 2-mercaptoacetate | Pentaerythritol tetrakis (3-mercaptopropionate) | 2,3-bis((2-mercaptoethyl)thio)-1-propanethiol |
| Polythiol amount | 36.9 % | 48.41 % | 40.01 % |
| % sulfur | 31.0 | 25.4 | 39.3 |
| Refractive index | 1.63 | 1.60 | 1.66 |

| Example | 4 | 5 | 6 |
|---|---|---|---|
| Alkyne thiol | 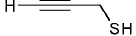 | 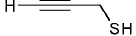 | 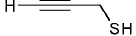 |
| Alkyne thiol amount | 48.65 % | 37.12 % | 45.37 % |
| Polythiol | 2-mercaptoethyl 2-mercaptoacetate | Pentaerythritol tetrakis (3-mercaptopropionate) | 2,3-bis((2-mercaptoethyl)thio)-1-propanethiol |
| Polythiol amount | 51.35 % | 62.88 % | 54.63 % |
| % sulfur | 43.17 | 32.94 | 53.68 |
| Refractive index | 1.67 | 1.63 | 1.71 |

| Example | 7 | 8 | 9 |
|---|---|---|---|
| Alkyne thiol | 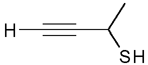 | 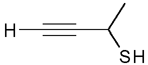 | 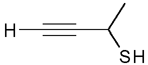 |
| Alkyne thiol amount | 53.09 % | 41.36 % | 49.80 % |
| Polythiol | 2-mercaptoethyl 2-mercaptoacetate | Pentaerythritol tetrakis (3-mercaptopropionate) | 2,3-bis((2-mercaptoethyl)thio)-1-propanethiol |
| Polythiol amount | 46.91 % | 58.64 % | 50.20 % |
| % sulfur | 39.44 | 30.71 | 49.33 |
| Refractive index | 1.66 | 1.63 | 1.70 |

| Example | 10 | 11 | 12 |
|---|---|---|---|
| Alkyne thiol | 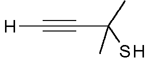 | 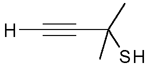 | 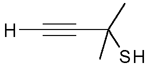 |
| Alkyne thiol amount | 56.82 % | 45.06 % | 53.57 % |
| Polythiol | 2-mercaptoethyl 2-mercaptoacetate | Pentaerythritol tetrakis (3-mercaptopropionate) | 2,3-bis((2-mercaptoethyl)thio)-1-propanethiol |
| Polythiol amount | 43.18 % | 54.94 % | 46.43 % |
| % sulfur | 36.30 | 28.78 | 45.62 |
| Refractive index | 1.65 | 1.62 | 1.68 |

| Example | 13 | 14 | 15 |
|---|---|---|---|
| Alkyne thiol | 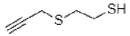 | 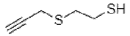 | 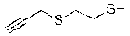 |
| Alkyne thiol amount | 63.47 % | 51.98 % | 60.36 % |
| Polythiol | 2-mercaptoethyl 2-mercaptoacetate | Pentaerythritol tetrakis (3-mercaptopropionate) | 2,3-bis((2-mercaptoethyl)thio)-1-propanethiol |
| Polythiol amount | 36.53 % | 48.02 % | 39.64 % |
| % sulfur | 46.07 | 37.73 | 53.55 |
| Refractive index | 1.68 | 1.65 | 1.71 |

| Example | 16 | 17 | 18 |
|---|---|---|---|
| Alkyne thiol | 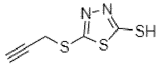 | 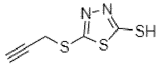 | 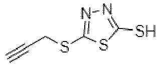 |
| Alkyne thiol amount | 71.21 % | 60.65 % | 68.44 % |
| Polythiol | 2-mercaptoethyl 2-mercaptoacetate | Pentaerythritol tetrakis (3-mercaptopropionate) | 2,3-bis((2-mercaptoethyl)thio)-1-propanethiol |
| Polythiol amount | 28.79 % | 39.35 % | 31.56 % |
| % sulfur | 48.41 | 41.23 | 54.27 |
| Refractive index | 1.69 | 1.67 | 1.72 |

## Claims

1. An optical material having a refractive index higher than or equal to 1.50 obtained by polymerization of a polymerizable composition comprising at least one polythiol and at least one polymerizable compound of formula (I): 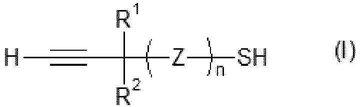
in which R¹ and R² represent, independently of each other, a hydrogen atom, a substituted or unsubstituted alkyl group, an aryl group, or R¹ and R², taken together, form a divalent group of formula -R¹-R²-, in which -R¹-R²- represents a substituted or unsubstituted alkylene group,
Z represents a divalent group connected to the adjacent mercapto -SH group through a carbon atom, and n = 0 or 1.

2. The optical material of claim 1, wherein Z is selected from substituted or unsubstituted:
- (hetero)alkylene groups, (hetero)arylene groups,
- carbonyl(hetero)alkylene groups, carbonyl(hetero)arylene groups,
- oxycarbonyl(hetero)alkylene groups, oxycarbonyl(hetero)arylene groups,
- carbonyloxy(hetero)alkylene groups, carbonyloxy(hetero)arylene groups,
- oxy(hetero)alkylene groups, oxy(hetero)arylene groups,
- thio(hetero)alkylene groups, thio(hetero)arylene groups,
- sulfo(hetero)alkylene groups, sulfo(hetero)arylene groups,
- amino(hetero)alkylene groups, amino(hetero)arylene groups,
- alkylamino(hetero)alkylene groups, alkylamino(hereto)arylene groups,
- arylamino(hetero)alkylene groups, arylamino(hetero)arylene groups,
- aminocarbonyl(hetero)alkylene groups, aminocarbonyl(hetero)arylene groups,
- alkylaminocarbonyl(hetero)alkylene groups, alkylaminocarbonyl(hereto)arylene groups,
- arylaminocarbonyl(hetero)alkylene groups, arylaminocarbonyl(hetero)arylene groups,
- carbonylamino(hetero)alkylene groups, carbonylamino(hetero)arylene groups,
- carbonylalkylamino(hetero)alkylene groups, carbonylalkylamino(hereto)arylene groups,
- carbonylarylamino(hetero)alkylene groups, carbonylarylamino(hetero)arylene groups, and combinations of the above divalent groups.

3. The optical material of any one of the preceding claims, wherein Z is selected from thio(hetero)arylene groups, thio(hetero)alkylene groups, and oxycarbonyl(hetero)alkylene groups, the (hetero)alkylene groups being substituted or unsubstituted.

4. The optical material of any one of the preceding claims, wherein said polymerizable compound is selected from the compounds of formula (I): 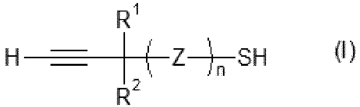 in which R¹ and R² represent, independently of each other, a hydrogen atom or a substituted or unsubstituted alkyl group, Z represents a thio(hetero)arylene group, a thio(hetero)alkylene group, or a poly[thio(hetero)alkylene] group, the (hetero)alkylene and (hetero)arylene groups being substituted or unsubstituted, and n = 0 or 1.

5. The optical material of any one of claims 1 to 2, wherein said polymerizable compound is selected from the compounds of formula (III): 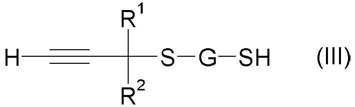 in which R¹ and R² represent, independently of each other, a hydrogen atom or a substituted or unsubstituted alkyl group, and G represents a divalent group connected to the adjacent mercapto -SH group through a carbon atom.

6. The optical material of any one of claims 1 to 2, wherein said polymerizable compound is selected from the compounds of formula (II): 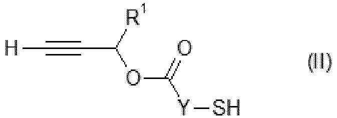 in which R¹ represents a hydrogen atom, a substituted or unsubstituted alkyl group, or an aryl group, Y represents an (hetero)alkylene group.

7. The optical material of any one of the preceding claims, wherein the alkyl and/or (hetero)alkylene groups comprise from 1 to 5 carbon atoms.

8. The optical material of any one of the preceding claims, wherein said polythiol is a compound of formula: R³(SH)ₙ₁ (IV)
wherein n1 represents an integer ranging from 2 to 6 and R³ represents an aliphatic, alicyclic, heterocyclic or aromatic group.

9. The optical material of any one of the preceding claims, wherein said polythiol is selected from the group consisting of pentaerythritol tetrakis(3-mercaptopropionate), pentaerythritol tetrakis(2-mercaptoacetate), tris(3-mercaptopropionate) trimethylolpropane, tris(2-mercaptoacetate) trimethylolpropane, 2,3-bis((2-mercaptoethyl)thio)-1-propanethiol, ethylene glycol bis (3-mercaptopropionate), butylene glycol bis (3-mercaptopropionate), 2-mercaptoethyl 2-mercaptoacetate, 2-mercaptoethyl 3-mercaptopropionate, 5,7-dimercaptomethyl-1,11-dimercapto-3,6,9-trithiaundecane, 4,7-dimercaptomethyl-1,11-dimercapto-3,6,9-trithiaundecane, 4,8-dimercaptomethyl-1,11-dimercapto-3,6,9-trithiaundecane, 2,5-bis (mercaptomethyl)-1,4-dithiane, bis (mercaptoethyl) sulfide, 1,1,3,3-tetrakis (mercaptomethylthio) propane, 4,6-bis (mercaptomethylthio)-1,3-dithiane, 2-(2,2-bis (mercaptomethylthio) ethyl)-1,3-dithietane, 1,1,2,2-tetrakis (mercaptomethylthio) ethane, 3-mercaptomethyl-1,5-dimercapto-2,4-dithiapentane, tris (mercaptomethylthio) methane, dipentaerythritol hexakis(3-mercaptopropionate), tris[2-(3-mercaptopropionyloxy)ethyl] isocyanurate, and ethanedithiol.

10. The optical material of any one of the preceding claims, further defined as having a refractive index higher than or equal to 1.60, more preferably higher than or equal to 1.65, even more preferably higher than or equal to 1.70.

11. The optical material of any one of the preceding claims, further defined as having a glass transition temperature higher than or equal to 85°C.

12. The optical material of any one of the preceding claims, further defined as the substrate of an optical lens.

13. The optical material of any one of the preceding claims, wherein said polymerizable compound is selected from the compounds of formulae:

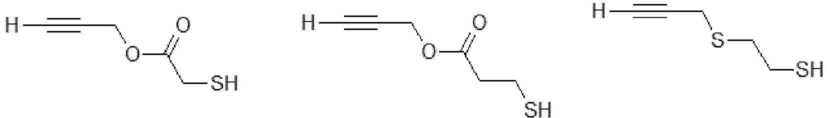

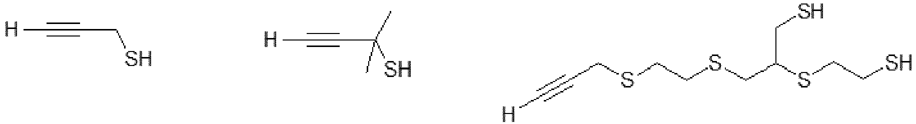

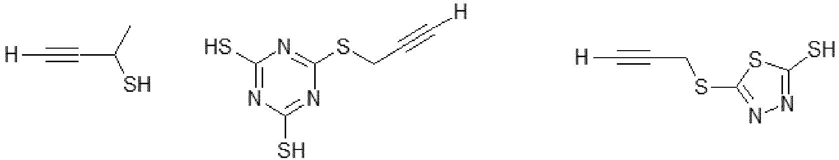

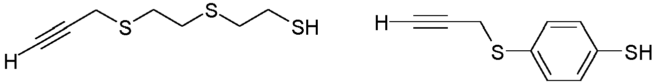

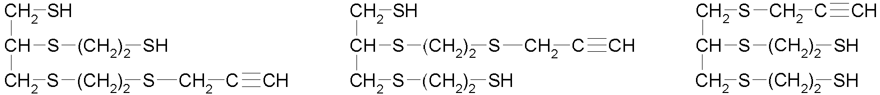

14. A process for the preparation of an optical material according to any one of the preceding claims, comprising polymerization of said polymerizable composition in the presence of at least one initiator, followed by a thermal post-cure.

15. A polymerizable compound selected from the compounds of formulae:

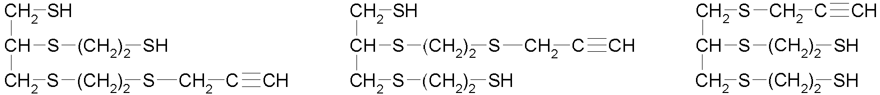

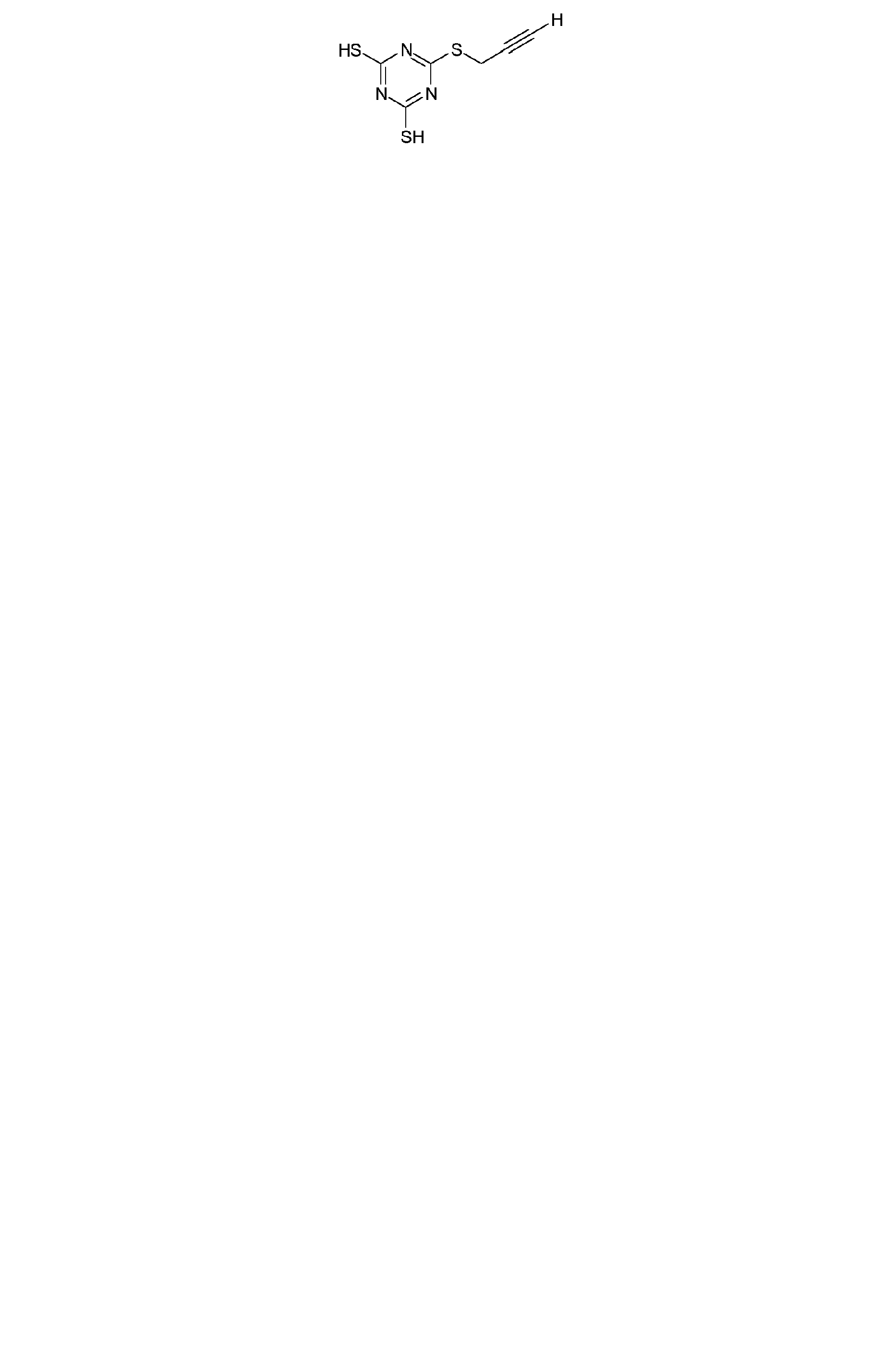

## Patentansprüche

1. Optisches Material mit einem Brechungsindex größer oder gleich 1,50, das erhalten wird durch Polymerisation einer polymerisierbaren Zusammensetzung, die mindestens ein Polythiol und mindestens eine polymerisierbare Verbindung der Formel (I) umfasst: 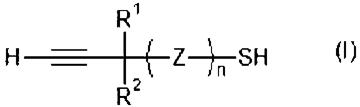
worin R¹ und R² unabhängig voneinander ein Wasserstoffatom, eine substituierte oder unsubstituierte Alkylgruppe, eine Arylgruppe repräsentieren, oder R¹ und R² zusammen eine zweiwertige Gruppe der Formel -R¹-R²-bilden, wobei -R¹-R²- eine substituierte oder unsubstituierte Alkylengruppe repräsentiert;
Z eine zweiwertige Gruppe repräsentiert, die mit der benachbarten Mercaptogruppe -SH durch ein Kohlenstoffatom verbunden ist, und n = 0 oder 1.

2. Optisches Material nach Anspruch 1, wobei Z ausgewählt ist aus substituierten oder unsubstituierten:
- (Hetero)alkylengruppen, (Hetero)arylengruppen,
- Carbonyl(hetero)alkylengruppen, Carbonyl(hetero)arylengruppen,
- Oxycarbonyl(hetero)alkylengruppen, Oxycarbonyl(hetero)arylengruppen,
- Carbonyloxy(hetero)alkylengruppen, Carbonyloxy(hetero)arylengruppen,
- Oxy(hetero)alkylengruppen, Oxy(hetero)arylengruppen,
- Thio(hetero)alkylengruppen, Thio(hetero)arylengruppen,
- Sulfo(hetero)alkylengruppen, Sulfo(hetero)arylengruppen,
- Amino(hetero)alkylengruppen, Amino(hetero)arylengruppen,
- Alkylamino(hetero)alkylengruppen, Alkylamino(hereto)arylengruppen,
- Arylamino(hetero)alkylengruppen, Arylamino(hetero)arylengruppen,
- Aminocarbonyl(hetero)alkylengruppen, Aminocarbonyl(hetero)arylengruppen,
- Alkylaminocarbonyl(hetero)alkylengruppen, Alkylaminocarbonyl(hereto)arylengruppen,
- Arylaminocarbonyl(hetero)alkylengruppen, Arylaminocarbonyl(hetero)arylengruppen,
- Carbonylamino(hetero)alkylengruppen, Carbonylamino(hetero)arylengruppen,
- Carbonylalkylamino(hetero)alkylengruppen, Carbonylalkylamino(hereto)arylengruppen,
- Carbonylarylamino(hetero)alkylengruppen, Carbonylarylamino(hetero)arylengruppen, und Kombinationen der obigen zweiwertigen Gruppen.

3. Optisches Material nach einem der vorhergehenden Ansprüche, wobei Z ausgewählt ist aus Thio(hetero)arylengruppen, Thio(hetero)alkylengruppen und Oxycarbonyl(hetero)alkylengruppen, wobei die (Hetero)alkylengruppen substituiert oder unsubstituiert sind.

4. Optisches Material nach einem der vorhergehenden Ansprüche, wobei die polymerisierbare Verbindung ausgewählt ist aus den Verbindungen der Formel (I): 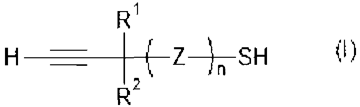 worin R¹ und R² unabhängig voneinander ein Wasserstoffatom oder eine substituierte oder unsubstituierte Alkylgruppe repräsentieren, Z eine Thio(hetero)arylengruppe, eine Thio(hetero)alkylengruppe oder eine Poly[thio(hetero)alkylen]-Gruppe repräsentiert, wobei die (Hetero)alkylen- und (Hetero)arylengruppen substituiert oder unsubstituiert sind und n = 0 oder 1.

5. Optisches Material nach einem der Ansprüche 1 bis 2, wobei die polymerisierbare Verbindung ausgewählt ist aus den Verbindungen der Formel (III): 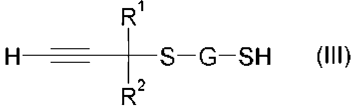 worin R¹ und R² unabhängig voneinander ein Wasserstoffatom oder eine substituierte oder unsubstituierte Alkylgruppe repräsentieren, und G eine zweiwertige Gruppe repräsentiert, die durch ein Kohlenstoffatom mit der benachbarten Mercaptogruppe -SH verbunden ist.

6. Optisches Material nach einem der Ansprüche 1 bis 2, wobei die polymerisierbare Verbindung ausgewählt ist aus den Verbindungen der Formel (II): 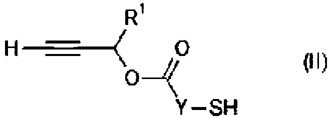 worin R¹ ein Wasserstoffatom, eine substituierte oder unsubstituierte Alkylgruppe oder eine Arylgruppe repräsentiert, Y eine (Hetero)alkylengruppe repräsentiert.

7. Optisches Material nach einem der vorhergehenden Ansprüche, wobei die Alkyl- und/oder (Hetero)alkylengruppen 1 bis 5 Kohlenstoffatome umfassen.

8. Optisches Material nach einem der vorangehenden Ansprüche, wobei das Polythiol eine Verbindung der folgenden Formel ist: R³(SH)ₙ₁ (IV)
wobei n1 eine ganze Zahl im Bereich von 2 bis 6 repräsentiert und R³ eine aliphatische, alicyclische, heterocyclische oder aromatische Gruppe repräsentiert.

9. Optisches Material nach einem der vorhergehenden Ansprüche, wobei das Polythiol ausgewählt ist aus der Gruppe bestehend aus Pentaerythrittetrakis(3-mercaptopropionat), Pentaerythrittetrakis(2-mercaptoacetat), Tris(3-mercaptopropionat)trimethylolpropan, Tris(2-mercaptoacetat)trimethylolpropan, 2,3-Bis((2-mercaptoethyl)thio)-1-propanthiol, Ethylenglykolbis(3-mercaptopropionat), Butylenglykolbis(3-mercaptopropionat), 2-Mercaptoethyl-2-mercaptoacetat, 2-Mercaptoethyl-3-mercaptopropionat, 5,7-Dimercaptomethyl-1,11-dimercapto-3,6,9-trithiaundecan, 4,7-Dimercaptomethyl-1,11-dimercapto-3,6,9-trithiaundecan, 4,8-Dimercaptomethyl-1,11-dimercapto-3,6,9-trithiaundecan, 2,5-Bis(mercaptomethyl)-1,4-dithian, Bis(mercaptoethyl)sulfid, 1,1,3,3-Tetrakis(mercaptomethylthio)propan, 4,6-Bis(mercaptomethylthio)-1,3-dithian, 2-(2,2-Bis(mercaptomethylthio)ethyl)-1,3-dithietan, 1,1,2,2-Tetrakis(mercaptomethylthio)ethan, 3-Mercaptomethyl-1,5-dimercapto-2,4-dithiapentan, Tris(mercaptomethylthio)methan, Dipentaerythrithexakis(3-mercaptopropionat), Tris[2-(3-mercaptopropionyloxy)ethyl]isocyanurat und Ethandithiol.

10. Optisches Material nach einem der vorhergehenden Ansprüche, weiter definiert als einen Brechungsindex größer oder gleich 1,60, bevorzugter größer oder gleich 1,65, noch bevorzugter größer oder gleich 1,70 aufweisend.

11. Optisches Material nach einem der vorhergehenden Ansprüche, weiter definiert als eine Glasübergangstemperatur größer oder gleich 85 °C aufweisend.

12. Optisches Material nach einem der vorhergehenden Ansprüche, weiter definiert als Substrat einer optischen Linse.

13. Optisches Material nach einem der vorhergehenden Ansprüche, wobei die polymerisierbare Verbindung ausgewählt ist aus den Verbindungen mit den folgenden Formeln:

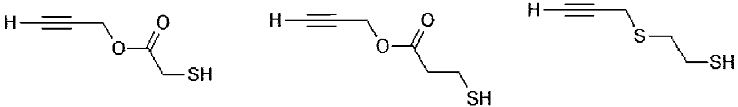

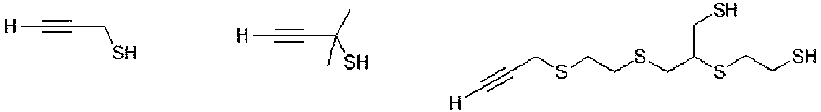

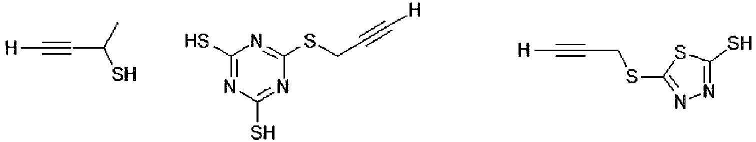

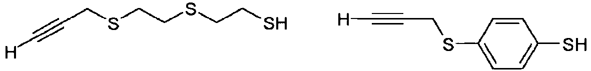

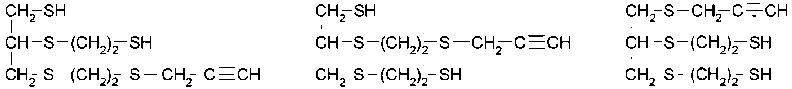

14. Verfahren zur Herstellung eines optischen Materials nach einem der vorhergehenden Ansprüche, umfassend Polymerisation der polymerisierbaren Zusammensetzung in Gegenwart mindestens eines Initiators, gefolgt von einer thermischen Nachhärtung.

15. Polymerisierbare Verbindung, ausgewählt aus den Verbindungen der Formeln:

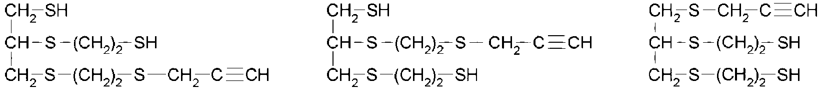

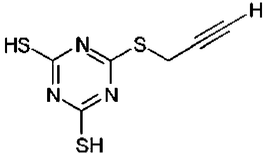

## Revendications

1. Matériau optique ayant un indice de réfraction supérieur ou égal à 1,50 obtenu par polymérisation d'une composition polymérisable comprenant au moins un polythiol et au moins un composé polymérisable de formule (I) : 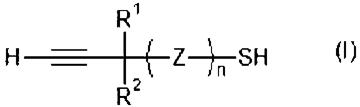
dans laquelle R¹ et R² représentent, indépendamment l'un de l'autre, un atome d'hydrogène, un groupe alkyle substitué ou non substitué, un groupe aryle, ou R¹ et R², pris ensemble, forment un groupe divalent de formule - R¹-R²-, dans laquelle -R¹-R²- représente un groupe alkylène substitué ou non substitué,
Z représente un groupe divalent relié au groupe mercapto -SH adjacent par l'intermédiaire d'un atome de carbone, et n = 0 ou 1.

2. Matériau optique selon la revendication 1, dans lequel Z est choisi parmi :
- groupes (hétéro)alkylène, groupes (hétéro)arylène,
- groupes carbonyl(hétéro)alkylène, groupes carbonyl(hétéro)arylène,
- groupes oxycarbonyl(hétéro)alkylène, groupes oxycarbonyl(hétéro)arylène,
- groupes carbonyloxy(hétéro)alkylène, groupes carbonyloxy(hétéro)arylène,
- groupes oxy(hétéro)alkylène, groupes oxy(hétéro)arylène,
- groupes thio(hétéro)alkylène, groupes thio(hétéro)arylène,
- groupes sulfo(hétéro)alkylène, groupes sulfo(hétéro)arylène,
- groupes amino(hétéro)alkylène, groupes amino(hétéro)arylène,
- groupes alkylamino(hétéro)alkylène, groupes alkylamino(hétéro)arylène,
- groupes arylamino(hétéro)alkylène, groupes arylamino(hétéro)arylène,
- groupes aminocarbonyl(hétéro)alkylène, groupes aminocarbonyl(hétéro)arylène,
- groupes alkylaminocarbonyl(hétéro)alkylène, groupes alkylaminocarbonyl(hétéro)arylène,
- groupes arylaminocarbonyl (hétéro)alkylène, groupes arylaminocarbonyl(hétéro)arylène,
- groupes carbonylamino(hétéro)alkylène, groupes carbonylamino(hétéro)arylène,
- groupes carbonylalkylamino(hétéro)alkylène, groupes carbonylalkylamino(hétéro)arylène,
- groupes carbonylarylamino(hétéro)alkylène, groupes carbonylarylamino(hétéro)arylène,
substitués ou non substitués, et des combinaisons des groupes divalents ci-dessus.

3. Matériau optique selon l'une quelconque des revendications précédentes, dans lequel Z est choisi parmi des groupes thio(hétéro)arylène, groupes thio(hétéro)alkylène et groupes oxycarbonyl(hétéro)alkylène, les groupes (hétéro)alkylène étant substitués ou non substitués.

4. Matériau optique selon l'une quelconque des revendications précédentes, dans lequel ledit composé polymérisable est choisi parmi les composés de formule (I) : 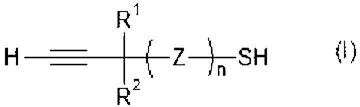 dans laquelle R¹ et R² représentent, indépendamment l'un de l'autre, un atome d'hydrogène ou un groupe alkyle substitué ou non substitué, Z représente un groupe thio(hétéro)arylène, un groupe thio(hétéro)alkylène ou un groupe poly[thio(hétéro)alkylène], les groupes (hétéro)alkylène et (hétéro)arylène étant substitués ou non substitués, et n = 0 ou 1.

5. Matériau optique selon l'une quelconque des revendications 1 à 2, dans lequel ledit composé polymérisable est choisi parmi les composés de formule (III) : 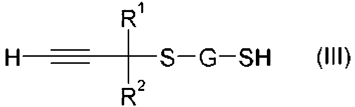 dans laquelle R¹ et R² représentent, indépendamment l'un de l'autre, un atome d'hydrogène ou un groupe alkyle substitué ou non substitué, et G représente un groupe divalent relié au groupe mercapto -SH adjacent par l'intermédiaire d'un atome de carbone.

6. Matériau optique selon l'une quelconque des revendications 1 à 2, dans lequel ledit composé polymérisable est choisi parmi les composés de formule (II) : 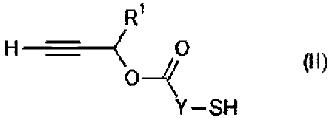 dans laquelle R¹ représente un atome d'hydrogène, un groupe alkyle substitué ou non substitué ou un groupe aryle, Y représente un groupe (hétéro)alkylène.

7. Matériau optique selon l'une quelconque des revendications précédentes, dans lequel les groupes alkyle et/ou (hétéro)alkylène comprennent de 1 à 5 atomes de carbone.

8. Matériau optique selon l'une quelconque des revendications précédentes, dans lequel ledit polythiol est un composé de formule : R³(SH)ₙ₁ (IV) dans laquelle n1 représente un entier allant de 2 à 6 et R³ représente un groupe aliphatique, alicyclique, hétérocyclique ou aromatique.

9. Matériau optique selon l'une quelconque des revendications précédentes, dans lequel ledit polythiol est choisi dans le groupe constitué par tétrakis(3-mercaptopropionate) de pentaérythritol, tétrakis(2-mercaptoacétate) de pentaérythritol, tris(3-mercaptopropionate) de triméthylolpropane, tris(2-mercaptoacétate) de triméthylolpropane, 2,3-bis((2-mercaptoéthyl)thio)-1-propanethiol, bis(3-mercaptopropionate) d'éthylène glycol, bis(3-mercaptopropionate) de butylène glycol, 2-mercaptoacétate de 2-mercaptoéthyle, 3-mercaptopropionate de 2-mercaptoéthyle, 5,7-dimercaptométhyl-1,11-dimercapto-3,6, 9-trithiaundécane, 4,7-dimercaptométhyl-1,11-dimercapto-3,6,9-trithiaundécane, 4,8-dimercaptométhyl-1,11-dimercapto-3,6,9-trithiaundécane, 2,5-bis(mercaptométhyl)-1,4-dithiane, bis(mercaptoéthyl)sulfure, 1,1,3,3-tétrakis(mercaptométhylthio)propane, 4,6-bis(mercaptométhylthio)-1,3-dithiane, 2-(2,2-bis(mercaptométhylthio)éthyl)-1,3-dithiétane, 1,1,2,2-tétrakis(mercaptométhylthio)éthane, 3-mercaptométhyl-1,5-dimercapto-2,4-dithiapentane, tris(mercaptométhylthio)méthane, hexakis(3-mercaptopropionate) de dipentaérythritol, isocyanurate de tris[2-(3-mercaptopropionyloxy)éthyle] et éthanedithiol.

10. Matériau optique selon l'une quelconque des revendications précédentes, défini en outre comme ayant un indice de réfraction supérieur ou égal à 1,60, plus préférablement supérieur ou égal à 1,65, encore plus préférablement supérieur ou égal à 1,70.

11. Matériau optique selon l'une quelconque des revendications précédentes, défini en outre comme ayant une température de transition vitreuse supérieure ou égale à 85 °C.

12. Matériau optique selon l'une quelconque des revendications précédentes, défini en outre en tant que le substrat d'une lentille optique.

13. Matériau optique selon l'une quelconque des revendications précédentes, dans lequel ledit composé polymérisable est choisi parmi les composés de formules :

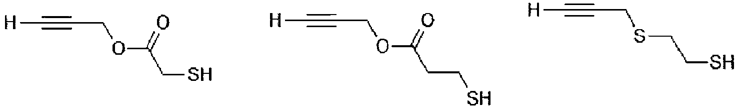

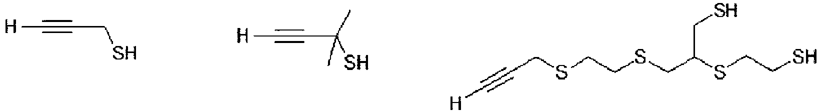

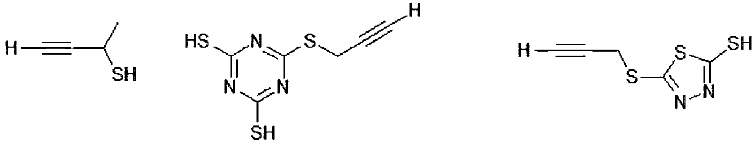

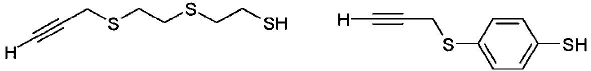

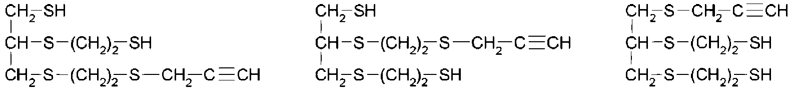

14. Procédé de préparation d'un matériau optique selon l'une quelconque des revendications précédentes, comprenant la polymérisation de ladite composition polymérisable en présence d'au moins un initiateur, suivie par un post-durcissement thermique.

15. Composé polymérisable choisi parmi les composés des formules :
